# FASCICULE DE BREVET EUROPEEN

(11) **EP 0 719 411 B1**
(45) Date de publication et mention de la délivrance du brevet: **03.03.1999**
(21) Numéro de dépôt: 94927694.3
(22) Date de dépôt: 16.09.1994
(51) Int. Cl.: G01N 33/00

(54) **PROCEDE ET APPAREIL DE DETECTION DES SUBSTANCES ODORANTES ET APPLICATIONS**
VERFAHREN UND VORRICHTUNG ZUM NACHWEIS VON SUBSTANZEN SOWIE ANWENDUNGEN
METHOD AND DEVICE FOR THE DETECTION OF ODOROUS SUBSTANCES AND APPLICATIONS

(30) Priorité: 17.09.1993 FR 9311291
(43) Date de publication de la demande: 03.07.1996
(73) Titulaire: ALPHA M.O.S., 31400 Toulouse (FR)
(72) Inventeur: MIFSUD, Jean, Christophe, F-31240 Saint-Jean (FR); MOY, Laurent, F-31400 Toulouse (FR)
(74) Mandataire: Orian, Yvette Suzanne
(86) Numéro de dépôt international: FR9401085
(87) Numéro de publication internationale: WO9508113

(56) Documents cités:
- EP-A- 0 239 233
- EP-A- 0 317 299
- EP-A- 0 398 687
- EP-A- 0 431 910
- WO-A-90/08314
- GB-A- 2 155 185
- TRAC, TRENDS IN ANALYTICAL CHEMISTRY, vol.11, no.2, Février 1992, AMSTERDAM, NL pages 61 - 67, XP247333 K. C. PERSAUD
- IEE PROCEEDINGS G. ELECTRONIC CIRCUITS & SYSTEMS, vol.137, no.3, Juin 1990, STEVENAGE GB pages 197 - 204, XP125837 H. V. SHURMER

## Description

La présente invention se rapporte aux domaines les plus variés, comme l'industrie alimentaire, l'industrie des cosmétiques, parfums et arômes, l'industrie chimique, l'industrie de l'emballage, l'environnement... et d'une façon plus générale à tous les domaines dans lesquels la détection des odeurs ou substances odorantes, ou substances volatiles, appelées indifféremment pour la suite de ce mémoire d'une façon générale substances odorantes ou odeurs, apporte des moyens d'investigation, de contrôle, et d'action sur ces domaines.

La détection des substances odorantes rencontre de nombreuses applications industrielles, notamment dans les processus industriels alimentaires, dans lesquels on peut par exemple déterminer le degré de fraîcheur et la qualité des produits grâce aux substances odorantes qu'ils dégagent. La chromatographie gazeuse, qui consiste en une méthode de détection sélective par séparation moléculaire des composés gazeux, est utilisée comme moyen de contrôle des odeurs. Toutefois, cette méthode comporte certains inconvénients; en effet, les résultats de détection des substances varient notamment selon la méthode d'échantillonnage choisie, le temps d'obtention des résultats est relativement long, et le coût d'un appareil de chromatographie gazeuse ajouté au coût d'un spectromètre de masse nécessaire à l'analyse des résultats est très onéreux.

Les développements récents dans le domaine de la détection des substances odorantes nous enseignent des procédés et appareils de détection possédant des capteurs gaz à semiconducteur, comme moyens de détection des substances odorantes. Les substances odorantes modifient les propriétés électriques des semiconducteurs en faisant varier leurs résistances électriques, et la mesure de ces variations permet de déterminer la concentration en substances odorantes. Ces procédés et appareils de détection des substances odorantes autorisent des temps de détection relativement brefs, de l'ordre de quelques secondes, comparés à ceux donnés par la chromatographie gazeuse, qui sont de l'ordre de quelques minutes à quelques heures. Par contre, les capteurs gaz à semiconducteur nécessitent de travailler à une haute température, jusqu'à 350°C. De plus, ces capteurs ont une bonne sensibilité mais une sélectivité moyenne à l'égard des substances odorantes.

D'autres technologies récentes de capteurs gaz sont maintenant utilisées, entraînant d'autres procédés et appareils de détection des substances odorantes. On rencontre par exemple des appareils possédant des capteurs gaz à polymère conducteur, et des appareils possédant des capteurs gaz à onde acoustique de surface.

Les capteurs à polymère conducteur possèdent un film constitué d'un polymère conducteur sensible aux molécules des substances odorantes, au contact desquelles la résistance électrique de ces capteurs est modifiée. La mesure de la variation de cette résistance permet de déterminer la concentration en substances odorantes. Un avantage de ce type de capteur est de fonctionner à des températures voisines de la température ambiante. On peut aussi obtenir, selon le polymère conducteur choisi, des sensibilités différentes pour détecter des substances odorantes différentes. D'une manière générale, on peut noter que les capteurs à polymère conducteur possèdent une meilleure sélectivité mais sont moins sensibles que les capteurs à semiconducteur.

Les capteurs gaz à onde acoustique de surface comprennent généralement un substrat ayant des caractéristiques piézo-électriques revêtu d'un film de polymère apte à adsorber des substances odorantes. La variation de masse résultante entraîne une variation de sa fréquence de résonance. Ce type de capteur permet de très bonnes mesures massiques des substances odorantes.

On connaît par exemple le document GB 2 155 185 qui se rapporte à un appareil et un procédé pour la détection et l'identification d'un composant aléatoire dans l'air ou dans un autre gaz. L'appareil comprend une pluralité de capteurs gaz d'une même technologie montés en série ou en parallèle, une pompe destinée à assurer une convection forcée des gaz sur les capteurs pendant un cycle de mesures déterminé. Chaque capteur est placé dans une enceinte distincte, et possède une réponse électrique différente à un même gaz. De manière alternative, ce document indique que plusieurs types de capteurs, de préférence électrochimiques, peuvent être combinées.

On connaît également le document EP-A-0 431 910 qui se rapporte à un dispositif pour la détection de la présence d'un produit chimique parmi plusieurs produits, qui comprend un groupe de détecteurs pouvant être des capteurs Tagushi, des capteurs à polymère conducteur, ou des capteurs à onde acoustique de surface. Les résultats des mesures (vecteur empreinte) sont enregistrés à des fins de comparaison et d'identification de produits, et peuvent être traités par un réseau de neurones.

Les procédés et appareils de détection des substances odorantes que l'on rencontre comprennent en général plusieurs capteurs afin d'augmenter la sélectivité des substances odorantes mais tous les capteurs utilisent un même (typede) technologie, c'est à dire semiconducteur ou polymère conducteur ou onde acoustique de surface. De ce fait les possibilités de détection des substances odorantes sont limitées notamment à la sensibilité des capteurs données par une seule technologie.

La présente invention permet de remédier à ces inconvénients, plus précisément elle consiste en un procédé de détection des substances odorantes ou volatiles comprenant les étapes suivantes : munir au moins une première et une deuxième enceinte respectivement de premiers et deuxièmes moyens de détection, lesdits premiers et deuxièmes moyens de détection possédant des propriétés électriques ou piézo-électriques sensibles aux molécules desdites substances odorantes ou volatiles; tester les propriétés électriques ou piézo-électriques de chacun desdits premiers et deuxièmes moyens de détection, ceux-ci étant en contact avec un débit variable contrôlé de gaz, pour effectuer une initialisation desdits premiers et deuxièmes moyens de détection avant la présence desdites substances odorantes ou volatiles; transporter lesdites substances odorantes ou volatiles d'un échantillon dans lesdites première et deuxième enceintes au moins, dans ledit débit variable contrôlé de gaz; mesurer les propriétés électriques ou piézo-électriques, pendant un intervalle de temps déterminé, de chacun desdits premiers et deuxièmes moyens de détection en présence dudit débit variable contrôlé de gaz comportant lesdites substances odorantes ou volatiles dudit échantillon, relativement à ladite initialisation, pour obtenir des données qualitatives et quantitatives sur lesdites substances odorantes ou volatiles dudit échantillon, comparer lesdites données au moins qualitatives dudit échantillon avec un fichier de substances odorantes ou volatiles, simultanément ou postérieurement aux mesures elles-mêmes, de manière à au moins identifier les substances odorantes ou volatiles détectées; caractérisé en ce qu'il comprend au moins les étapes suivantes : munir lesdits premiers moyens de détection de ladite première enceinte d'une pluralité de capteurs gaz à semiconducteur, ou d'une pluralité de capteurs gaz à polymère conducteur, ou d'une pluralité de capteurs gaz à onde acoustique de surface; munir lesdits deuxièmes moyens de détection de ladite deuxième enceinte d'une pluralité de capteurs gaz à semiconducteur, ou d'une pluralité de capteurs gaz à polymère conducteur, ou d'une pluralité de capteurs gaz à onde acoustique de surface, lesdits premiers et deuxièmes moyens de détection étant munis respectivement de capteurs gaz de types de technologie différentes; nettoyer lesdits premiers et deuxièmes moyens de détection par un balayage de ces derniers avec un gaz, pour initialiser lesdits premiers et deuxièmes moyens de détection.

Le transport des substances odorantes d'un échantillon dans un débit variable contrôlé de gaz, permet de réaliser des détections très rapides de substances odorantes, de l'ordre de quelques secondes. De plus, cette caractéristique permet de se rapprocher des conditions de transport des substances odorantes dans un nez biologique, et permet d'assurer la reproductibilité des procédés de détection selon l'invention en contrôlant les conditions de transport des substances odorantes, ce qui n'est pas le cas dans les procédés de l'art antérieur avec des moyens de convection naturelle non contrôlés. Les moyens utilisés pour réaliser ledit débit variable contrôlé de gaz sont avantageusement constitués d'au moins une pompe à débit variable, ladite pompe étant également avantageusement utilisée pour établir un débit variable contrôlé de gaz pendant le test des propriétés électriques et/ou piézo-électriques et le nettoyage des moyens de détection. Le gaz utilisé peut être l'air ambiant, purifié le cas échéant. Pendant la phase de mesure des propriétés électriques et/ou piézo-électriques, le débit de gaz peut être diminué ou augmenté selon que les substances odorantes deviennent respectivement plus ou moins volatiles tout au long de l'intervalle de temps de mesure.

Selon une caractéristique avantageuse, le procédé selon l'invention consiste à munir une troisième enceinte de troisièmes moyens de détection, lesdits troisièmes moyens de détection comprenant une pluralité de capteurs gaz à semiconducteur, ou une pluralité de capteurs gaz à polymère conducteur, ou une pluralité de capteurs gaz à onde acoustique de surface.

L'intérêt de cette caractéristique est de munir le procédé selon l'invention de trois moyens de détection différents afin d'obtenir une sensibilité et une sélectivité générale de détection plus importante. En effet, comme cela a été développé en début de mémoire, les différents types de capteurs, à semiconducteur, à polymère conducteur, ou à onde acoustique de surface ont chacun des caractéristiques différentes qui se complètent : bonne sensibilité pour les capteurs à semiconducteur, bonne sélectivité pour les capteurs à polymère conducteur, bonne mesure massique pour les capteurs à onde acoustique de surface.

Selon une caractéristique avantageuse, le procédé selon l'invention comprend au moins un enregistrement desdites données qualitatives sur lesdites substances odorantes, à un instant déterminé, pour constituer un fichier de substances odorantes.

Cette caractéristique permet de réaliser un fichier, notamment par l'intermédiaire d'une unité de traitement de données, dans lequel sont enregistrées les données fournies par les mesures effectuées sur des substances odorantes connues, sous forme d'empreintes olfactives. Les substances odorantes sont caractérisées ainsi chacune par une empreinte olfactive qui lui est propre, ladite empreinte olfactive étant composée de données fournies par chacun des moyens de détection. Il faut relever que cette empreinte olfactive peut évoluer tout au long de l'intervalle de temps de mesure selon notamment le degré de volatilité des substances odorantes, ou la valeur du débit variable de gaz.

Un opérateur peut avantageusement visualiser les données des mesures par l'intermédiaire d'un moniteur connecté à l'unité de traitement de données, lesdites données pouvant être présentées sous toutes formes connues et largement répandues comme des histogrammes, des courbes, des nuages de points, des représentations en étoile, et plus généralement toutes formes de représentations bidimensionnelles ou tridimensionnelles.

Selon une caractéristique avantageuse, le procédé selon l'invention comprend au moins les étapes supplémentaires suivantes:
- transporter lesdites substances odorantes ou volatiles d'un deuxième échantillon dans lesdites première et deuxième enceintes au moins, dans un débit variable contrôlé de gaz,
- mesurer les propriétés électriques ou piézo-électriques, pendant un intervalle de temps déterminé, de chacun desdits premiers et deuxièmes moyens de détection en présence dudit débit variable contrôlé de gaz comportant lesdites substances odorantes ou volatiles, relativement à ladite initialisation, pour obtenir des données qualitatives et quantitatives sur lesdites substances odorantes ou volatiles,
- comparer lesdites données avec ledit fichier de substances odorantes ou volatiles, simultanément ou postérieurement aux mesures elles-mêmes, ladite comparaison s'effectuant au plus pendant la totalité dudit intervalle de temps de mesure, de manière à au moins identifier, au plus reconnaître au moyen d'un réseau de neurones, le cas échéant, les substances odorantes ou volatiles détectées.

La réalisation d'un fichier d'empreintes olfactives donne la possibilité d'introduire une étape de comparaison dans le procédé selon l'invention, pour la détection de substances odorantes de produits inconnus d'un opérateur. En effet, les données de mesure constituant une empreinte olfactive à un instant déterminé, peuvent être transmises avantageusement tout au long de l'intervalle de temps de mesure du produit inconnu, à l'unité de traitement de données qui compare l'empreinte aux empreintes olfactives présentes dans le fichier, et qui informe, par exemple au moyen d'un moniteur, l'opérateur que l'on est en présence, soit de substances odorantes connues donc présentes dans le fichier, soit de substances odorantes inconnues donc absentes du fichier. Dans le cas de substances odorantes reconnues par l'unité de traitement de données, l'identité desdites substances peut être communiquée à l'opérateur. Dans le cas contraire, l'unité de traitement informe l'opérateur de substances odorantes inconnues dans le fichier. Cette étape de comparaison et de reconnaissance des substances odorantes est réalisée au moyen d'un logiciel, avantageusement un logiciel de réseau neuronal.

L'opérateur peut choisir d'enregistrer la ou les substances inconnues et créer une nouvelle empreinte olfactive dans le fichier, par exemple dans le but d'identifier les mêmes substances ultérieurement. En effet, l'étape de comparaison peut être constituée d'une simple identification de substances odorantes inconnues, à partir d'une empreinte olfactive présente dans le fichier, cette étape de comparaison ne nécessitant pas l'utilisation d'un logiciel de réseau neuronal.

Il faut relever que l'étape de simple comparaison des empreintes olfactives apporte l'avantage pratique de pouvoir identifier deux ou plusieurs produits comme étant similaires car possédant des empreintes olfactives identiques.

Il faut noter que dans le cas ou la comparaison des données des mesures avec ledit fichier de substances odorantes se fait postérieurement aux mesures elles-mêmes, il est nécessaire d'enregistrer lesdites mesures.

Selon d'autres caractéristiques, le procédé de détection des substances odorantes suivant l'invention comprend des moyens de séparation desdites substances odorantes, lesdits moyens de séparation comprenant avantageusement un dispositif de chromatographie gazeuse.

Ces caractéristiques permettent d'obtenir une empreinte olfactive moléculaire des substances odorantes, lesdits moyens de séparation par chromatographie gazeuse intervenant avant lesdits moyens de détection.

Dans ce cas, le dispositif conventionnel de chromatographie gazeuse sépare préalablement les différentes molécules des substances odorantes, qui sont ensuite transportées par type de molécules vers les moyens de détection, lesdits moyens de détection permettent ensuite d'obtenir une empreinte olfactive pour chacune des molécules, suivant l'un des procédés selon l'invention décrit plus haut. Ce procédé selon l'invention permet notamment d'associer les avantages de la chromatographie gazeuse (séparation moléculaire) aux présents moyens de détection, de façon à constituer notamment un fichier d'empreinte olfactive moléculaire.

L'invention a aussi pour objet, un appareil de détection des substances odorantes ou volatiles permettant de mettre en oeuvre les procédés de détection des substances odorantes ou volatiles décrit ci-dessus, et des applications détaillées en fin de mémoire.

D'autres avantages et caractéristiques apparaîtront à la lecture de la description, de deux exemples de mode de réalisation d'un appareil de détection des substances odorantes ou volatiles selon l'invention, et de quelques exemples d'applications qui suivent, accompagnés des dessins annexés, exemples donnés à titre d'illustration sans qu'aucune interprétation restrictive de l'invention ne puisse en être tirée.

La Figure 1 représente un schéma fonctionnel d'un appareil de détection des substances odorantes ou volatiles selon l'invention comprenant quatre enceintes montées en séries.

La Figure 2 représente un schéma fonctionnel d'un appareil de détection des substances odorantes ou volatiles selon l'invention comprenant quatre enceintes montées en parallèles et en séries.

Les Figures 3 à 6 représentent quatre diagrammes d'empreintes de substances odorantes de deux cafés différents.

La Figure 7 montre un diagramme d'une empreinte olfactive de substances odorantes provenant d'un parfum.

La Figure 8 montre un diagramme d'une empreinte olfactive de substances odorantes provenant d'un parfum différent de celui utilisé sur la Figure 7.

Les Figures 9, 10 et 11 montrent des diagrammes, représentant les empreintes olfactives provenant respectivement d'un vin de table, de l'éthanol, et du bouquet dudit vin de table.

Les Figures 12, 13 et 14 montrent des diagrammes d'empreintes olfactives provenant de deux explosifs différents, sous des formes et des conditions d'expérience différentes.

Les Figures 15 et 16 montrent des diagrammes d'empreintes olfactives en fonction du temps provenant de deux bouchons en liège.

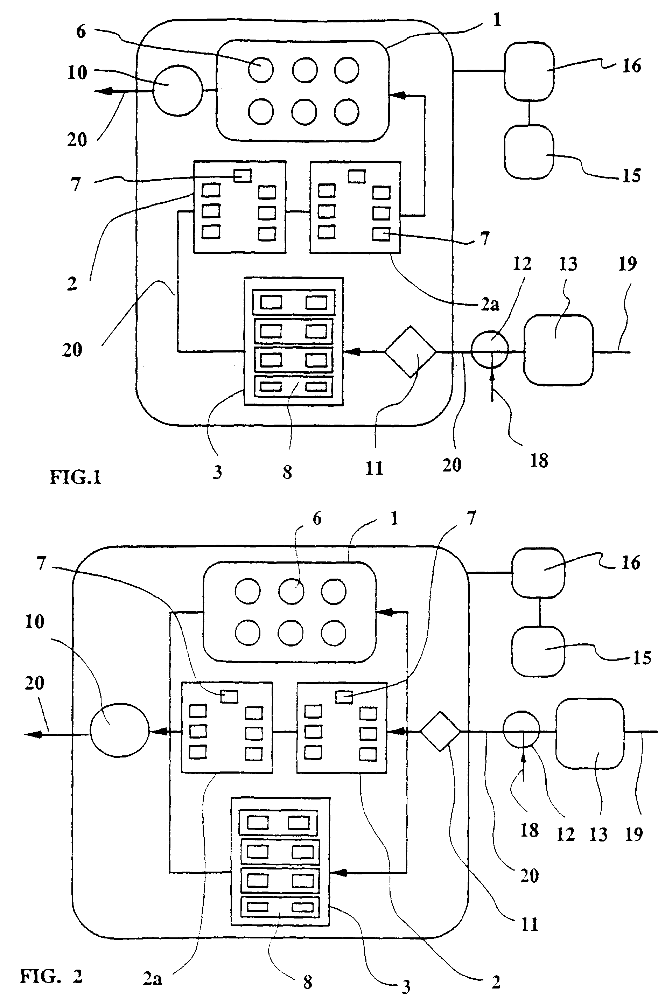

L'appareil de détection des substances odorantes représenté sur la Figure 1 comprend, une première 1, deuxième 2, une troisième 3, et une quatrième 2a enceinte munies respectivement de six capteurs gaz à semiconducteur 6, sept capteurs gaz à polymère conducteur 7, quatre capteurs gaz à onde acoustique de surface 8, sept capteurs gaz à polymère conducteur 7, une pompe à débit variable 10 pour fluides gazeux, une électronique de mesure 15, une unité de traitement de données 16.

Le nombre des capteurs 6, 7, 7, 8 respectif de chaque enceinte 1, 2, 2a, 3 est déterminé de manière à rendre maximales la sensibilité et la sélectivité de l'appareil à l'égard des substances odorantes à détecter.

Les quatre enceintes 1, 2, 3, 2a sont montées en séries sur une conduite 20 dans laquelle circule un débit de gaz généré par une pompe 10 à débit variable, lequel est placé en sortie de la dernière enceinte par rapport au sens de circulation du débit de gaz comme indiqué sur la Figure 1. Cette position de la pompe 10 est choisie de telle sorte que celle-ci ne contamine pas les capteurs équipant les différentes enceintes. De ce fait ladite pompe à débit variable établit le débit de gaz dans les quatre enceintes par aspiration. Les différents capteurs 6, 7, 8 sont placés dans les enceintes de façon à ce qu'ils soient évidemment en contact avec le débit de gaz. Le débit de gaz réglable sera avantageusement variable entre les valeurs de 5 ml/mn et 500 ml/mn dans le cas d'enceintes placées en séries.

L'ordre de placement des quatre enceintes 1, 2, 3, 2a sur la conduite 20 est avantageusement déterminé en fonction des perturbations provoquées par les capteurs de chaque enceinte sur le gaz transportant les substances odorantes à détecter, ou sur les substances odorantes elles-mêmes, et en fonction de leur sensibilité. En conséquence, dans le sens de circulation du débit de gaz, on rencontre tel que le montre la Figure 1, la troisième enceinte 3 munie de capteurs à onde acoustique de surface 8, les deuxième 2 et quatrième enceintes 2a munies de capteurs à polymère conducteur 7, et enfin la première enceinte 1 munie de capteurs à semiconducteur 6, ces derniers travaillant à haute température, modifiant donc le débit de gaz de façon plus importante.

Sur la conduite 20 à l'entrée de la troisième 1 enceinte, un filtre 11 assure une certaine pureté du gaz circulant dans la conduite 20 et les enceintes 1, 2, 2a, 3. Le filtre 11 préserve les capteurs 6, 7, 8 des contaminations provoquées par des impuretés présentes dans le gaz aspiré, par exemple dans l'air ambiant le cas échéant, mais autorise évidemment le passage des substances odorantes.

En amont du filtre 11 dans le sens de circulation du débit de gaz, et connectée sur la conduite 20, une vanne 12 à trois voies permet de sélectionner soit une aspiration du gaz à travers la conduite 18 reliée à l'air ambiant, soit une aspiration dans une chambre 13 pour échantillons comprenant des substances odorantes ou des produits dont on désire détecter les substances odorantes, ladite chambre étant reliée par une conduite 19 à l'air ambiant.

L'aspiration à travers la conduite 18 est notamment utilisée pour l'étape de test des propriétés électriques et/ou piézo-électriques des capteurs 6, 7, 8, et l'étape de nettoyage des capteurs gaz 6, 7, 8 après un passage des substances odorantes dans les enceintes 1, 2, 3, 2a, des procédés selon l'invention, le cas échéant.

L'aspiration à travers la conduite 19 est notamment utilisée pour l'étape de mesure des propriétés électriques et/ou piézo-électriques des capteurs gaz 6, 7, 8 en présence du débit variable contrôlé de gaz comportant les substances odorantes. Ainsi, à son passage dans la chambre 13 pour échantillons, l'air entraîne les substances odorantes, puis traverse le filtre 11, et respectivement les quatre enceintes 3, 2, 2a, 1 comportant les capteurs gaz 8, 7, 6 avant de traverser la pompe 10, et d'être rejeté à l'extérieur de l'appareil de détection. Il peut s'avérer nécessaire de chauffer le produit placé dans la chambre 13 afin de générer ou d'accélérer la production de substances odorantes, notamment si ledit produit est peu volatil. Dans ce cas, la chambre 13 comprend tous moyens de chauffage non polluant, comme par exemple un chauffage électrique par résistance.

Il faut remarquer que la présence de la chambre 13 et de la vanne 12 à trois voies n'est pas indispensable au fonctionnement de l'appareil de détection des substances odorantes selon l'invention. En effet, en l'absence de la chambre 13 et de la vanne 12, l'aspiration du débit de gaz ou du débit de gaz comportant les substances odorantes se fait directement à travers la conduite 20 d'entrée dans l'appareil ou directement à travers le filtre 11. Cette configuration (non représentée) de l'appareil suivant l'invention permet notamment la détection de substances odorantes présentes dans un gaz ambiant.

Un capteur au moins de température et un capteur au moins d'humidité (tous deux non représentés) pour mesurer respectivement la température du débit de gaz le taux d'humidité du débit de gaz, sont placés, dans au moins une des enceintes. En effet, les paramètres de température et de taux d'humidité influencent les mesures des propriétés électriques et/ou piézo-électriques des capteurs gaz 6, 7, 8, et de ce fait il est nécessaire de connaître la valeur de ces paramètres pour pouvoir effectuer des comparaisons de substances odorantes.

L'ensemble des capteurs gaz 6, 7, 8, et les capteurs de température et d'humidité sont connectés à une électronique de mesure 15 permettant, dans les procédés selon l'invention, l'étape de test des propriétés électriques et/ou piézo-électriques des capteurs gaz 6, 7, 8, la mesure de température et de taux d'humidité du débit de gaz, et l'étape de mesure des propriétés électriques et/ou piézo-électriques des capteurs gaz 6, 7, 8 en présence des substances odorantes.

L'électronique de mesure 15 permet d'appliquer indépendamment d'une part une tension électrique réglable aux bornes de chacun des capteurs 6, 7, d'autre part une onde acoustique de surface a chacun des capteurs 8, au moins pour l'étape de test et l'étape de mesure des propriétés électriques et/ou piézo-électriques desdits capteurs lors de l'exécution des procédés selon l'invention.
Le choix d'une tension électrique appliquée aux capteurs 6, 7, et le choix d'une fréquence appliquée aux capteurs 8, lors de l'étape de test en l'absence de substances odorantes mais en présence du débit de gaz, déterminent les valeurs d'initialisation desdits capteurs, par rapport auxquelles seront effectuées les mesures lors de l'étape de mesure des propriétés électriques et/ou piézo-électriques en présence du débit de gaz comportant les substances odorantes.

Chacun des capteurs gaz à semiconducteur 6, chacun des capteurs gaz à polymère conducteur 7, et chacun des capteurs gaz à onde acoustique de surface 8 donnent une information qualitative et quantitative sur les substances odorantes présentes dans les enceintes 1, 2, 2a, 3.

En ce qui concerne les capteurs gaz à semiconducteur 6 et les capteurs gaz à polymère conducteur 7, l'électronique de mesure 15 mesure les valeurs des variations de résistance, tension, ou conductance de ces capteurs comme données quantitatives de substances odorantes. En ce qui concerne les capteurs à onde acoustique de surface 8, l'électronique de mesure 15 mesure la variation de fréquence de résonance des capteurs induite par la masse de molécules de substances odorantes adsorbée par le revêtement de polymère du capteur, comme données quantitatives de substances odorantes présentes.

Les données qualitatives sont fournies par le choix des parties sensibles équipant les capteurs, notamment le choix du polymère pour les capteurs à polymère conducteur 7 et les capteurs à onde acoustique de surface 8, choix effectué en fonction des besoins d'un utilisateur de l'appareil. En ce qui concerne les capteurs à semiconducteur 6, les données qualitatives sont fournies par le choix préalable du type d'oxyde métallique ainsi que notamment le choix de leur température respective de fonctionnement, en fonction des besoins d'un utilisateur. La température est réglable indépendamment pour chaque capteur à semiconducteur 6 au moyen de l'électronique de mesure 15, lors de l'étape de test des propriétés électriques dans les procédés selon l'invention, afin de déterminer une plage d'utilisation optimale desdits capteurs, ces derniers réagissant à des substances odorantes différentes selon que leur température propre est plus ou moins élevée.

Il faut noter qu'à tous moments des procédés de détection des substances odorantes selon l'invention le débit de gaz peut être modifié de façon contrôlé grâce à la pompe à débit variable. Cette caractéristique permet, notamment d'accélérer les procédés de détection dans le cas de détection de substances odorantes de produits peu volatils, ou d'accélérer le temps de réponse des capteurs dans le cas de détection de substances odorantes en faible quantité.

L'unité de traitement de données 16 reçoit les données qualitatives et quantitatives sur les différentes substances odorantes détectées et mesurées qui lui sont transmis par l'électronique de mesure 15. Cette unité de traitement de données 16 comprend une unité centrale munie de logiciels d'applications (non représentée) et avantageusement un moniteur (non représenté) servant d'interface entre ladite unité centrale et un opérateur. L'unité centrale autorise, l'étape d'enregistrement des données des mesures effectuées par l'électronique de mesure 15, cet enregistrement permettant avantageusement de constituer un fichier de substances odorantes, et l'étape de comparaison des données des mesures avec ledit fichier de substances odorantes.

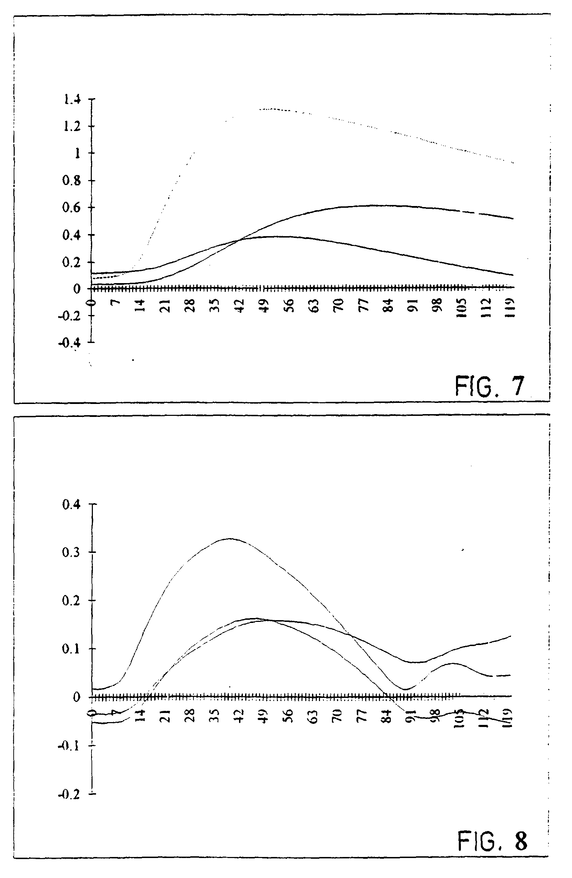

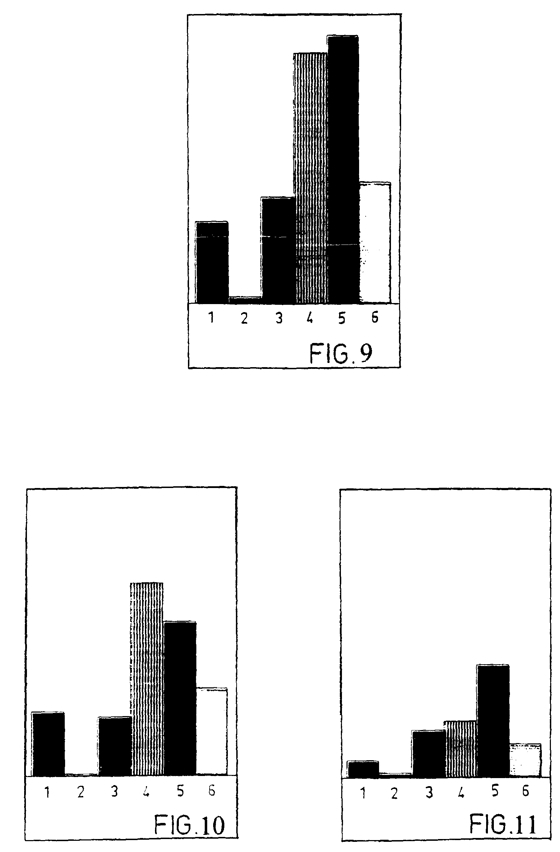

L'électronique de mesure 15 réalise notamment des mesures sur les capteurs 6, 7, 8 avantageusement de façon continue pendant un intervalle de temps déterminé par l'opérateur selon les besoins. Durant ledit intervalle de temps, l'unité de traitement de données 16 enregistre les données constituées par les résultats des mesures, de manière à fournir à l'opérateur des informations sur les substances odorantes présentes en fonction du temps. Cette caractéristique procure des avantages qui seront développés ultérieurement dans des exemples de détection de substances odorantes selon les procédés suivant l'invention, notamment dans l'exemple de détection de substances odorantes de parfums, illustré par les Figures 8 et 9.

Il faut remarquer que l'enregistrement des données permet à l'opérateur d'être informé, à tout instant de l'intervalle de temps de mesure, des valeurs de la mesure instantanée de tous les capteurs, l'ensemble de ces valeurs instantanées définissant alors une empreinte olfactive des substances odorantes à cet instant. Cette empreinte olfactive peut prendre toutes formes conventionnelles avantageusement sur un moniteur (histogramme, courbe, étoile, etc). L'évolution de l'empreinte olfactive au cours de l'intervalle de temps de mesure peut également être représentée sur le moniteur de toutes façons conventionnelles (courbes multiples, diagramme tridimensionnel, etc).

L'étape de comparaison des données des mesures avec le fichier de substances odorantes peut avoir lieu à tout instant au cours de l'intervalle de temps de mesure ou avoir lieu postérieurement aux mesures. Dans ce dernier cas, il est nécessaire de procéder à l'enregistrement des données de mesure pendant l'intervalle de temps de mesure.

L'étape de comparaison des données doit être différenciée suivant que l'on désire, identifier deux ou plusieurs substances odorantes, ou reconnaître des substances odorantes.

En ce qui concerne l'identification de deux ou plusieurs substances odorantes, il suffit d'avoir placé dans le fichier, lors d'une première détection au moins, une ou plusieurs empreintes olfactives par rapport auxquelles on désire comparer d'autres substances odorantes. Il faut noter que l'opérateur n'a pas dans ce cas nécessairement besoin de connaître l'identité des substances odorantes placées dans le fichier sous forme d'empreintes olfactives. Ultérieurement, lors des détections suivantes, dans le but de retrouver la ou les substances odorantes enregistrées, l'opérateur comparera les nouvelles empreintes, obtenues comme indiqué dans les paragraphes précédents, avec celles enregistrées, et effectuera l'identification, le cas échéant.

Il faut indiquer que des substances odorantes identiques peuvent avoir été mesurées ou enregistrées selon des données quantitatives ou des concentration différentes, ce qui rend la comparaison des empreintes olfactives plus difficile, celles-ci étant différentes malgré l'identité des substances odorantes. De façon à obtenir des empreintes indépendantes de la concentration, l'unité de traitement de données 16 peut effectuer une normalisation des données. C'est à dire ramener toutes les données des capteurs définissant une empreinte olfactive, en proportions égales, jusqu'à une valeur unitaire que l'on fixe avantageusement sur le capteur fournissant la plus forte donnée.

La reconnaissance des substances odorantes par l'appareil selon l'invention, est possible à l'aide de moyens supplémentaires par rapport à la simple identification. En effet la reconnaissance des substances odorantes entraîne que l'appareil possède, d'une part une mémoire de toutes les substances odorantes à reconnaître, et d'autre part des moyens d'appréciation, c'est à dire d'identification ou de différenciation des substances odorantes possédant des empreintes olfactives ressemblantes, similaires, ou différentes.

Dans un premier temps, il est donc nécessaire de placer des empreintes olfactives de substances odorantes, avantageusement dans une mémoire de l'unité centrale de l'unité de traitement de données 16, afin de créer un fichier de substances odorantes connues. L'opérateur associe alors à une ou plusieurs empreintes olfactives le nom de la substance odorante correspondante, dans la mémoire de l'unité centrale. Lesdits moyens d'appréciation sont fournis par un logiciel de réseau neuronal qui traite les données de mesure de chacun des capteurs définissant une empreinte olfactive pour les comparer aux données des empreintes olfactives présentes dans le fichier, et apprécier l'identification, ou la différenciation, ou encore l'absence des substances odorantes détectées par rapport aux substances odorantes présentes dans le fichier. L'opérateur est informé des résultats de la comparaison avantageusement par l'intermédiaire du moniteur qui peut par exemple afficher, le cas échéant, soit le nom de la substance odorante détectée, soit un message "substance odorante inconnue".

Une fois que l'opérateur a obtenu les résultats désirés, il est nécessaire de nettoyer les capteurs avant une autre utilisation.Cette opération est réalisée au moyen de la pompe 10 à débit variable, comme cela a été indiqué plus haut, par balayage des capteurs 6, 7, 8 avec un débit de gaz avantageusement supérieur jusqu'à ce que ces derniers retrouvent leur valeur d'initialisation respective donnée à l'étape de test des propriétés électriques et/ou piézo-électriques. L'électronique de mesure 15 permet de contrôler cette opération. Les capteurs à semiconducteur 6 peuvent être chauffés simultanément pour accélérer l'opération de nettoyage, notamment avec les mêmes moyens que pour le réglage de leur température lors de l'étape de test et d'optimisation des propriétés électriques desdits capteurs 6.

La Figure 2 représente une variante de l'appareil de détection des substances odorantes selon l'invention illustré par la Figure 1. Il s'en différencie par une autre disposition des quatre enceintes 1, 2, 2a, 3. En effet, les enceintes 1, 3, et le groupe des deux enceintes 2, 2a sont placées en parallèle sur la conduite 20, comme le montre la Figure 2. Il est à noter que les deux enceintes 2, 2a, comportant toutes deux des capteurs à polymère conducteur 7, sont placées en séries sur une des branches du montage en parallèle. L'intérêt d'une telle caractéristique est de rendre indépendant la traversée du débit du gaz d'une enceinte par rapport à une autre munie de capteurs d'une technologie différente. Ainsi, on évite les modifications éventuelles du débit de gaz d'une enceinte par rapport à la suivante dans un placement en série, modifications dues au passage du débit de gaz sur les capteurs. De plus, il existe dans ce cas la possibilité d'isoler, selon toutes méthodes connues, au moins une des branches parallèles de la conduite 20, donc d'isoler au moins une enceinte, si désiré, par exemple au moyen de vannes (non représentées).

La pompe 10 à débit variable utilisée sur l'appareil selon l'invention représenté sur la Figure 2 permettra, dans le cas d'enceintes placées en parallèle sur la conduite 20, de pouvoir disposer avantageusement d'un débit de gaz réglable compris entre les valeurs de 5 ml/mn et 500 ml/mn pour chaque enceinte.

L'appareil selon l'invention peut se présenter de façon compacte et portable, d'encombrement relativement réduit. En effet, les enceintes 1, 2, 2a, 3 peuvent posséder chacune un volume de l'ordre du cm³, suffisant pour la détection des substances odorantes. L'unité de traitement de données peut elle aussi être d'encombrement très réduit de l'ordre de celui d'une carte électronique. Le moniteur quand à lui peut être un écran à affichage à cristaux liquides ou à diodes, ou un écran cathodique de tailles réduites eux aussi. Ainsi, l'appareil peut aisément se transporter en tous lieux par une seule personne. Dans le cas de détection de substances odorantes présentes notamment dans l'air ambiant, la chambre 13, la vanne à trois voies 12 et les conduites 18, 19 ne sont pas indispensables comme cela a déjà été indiqué précédemment.

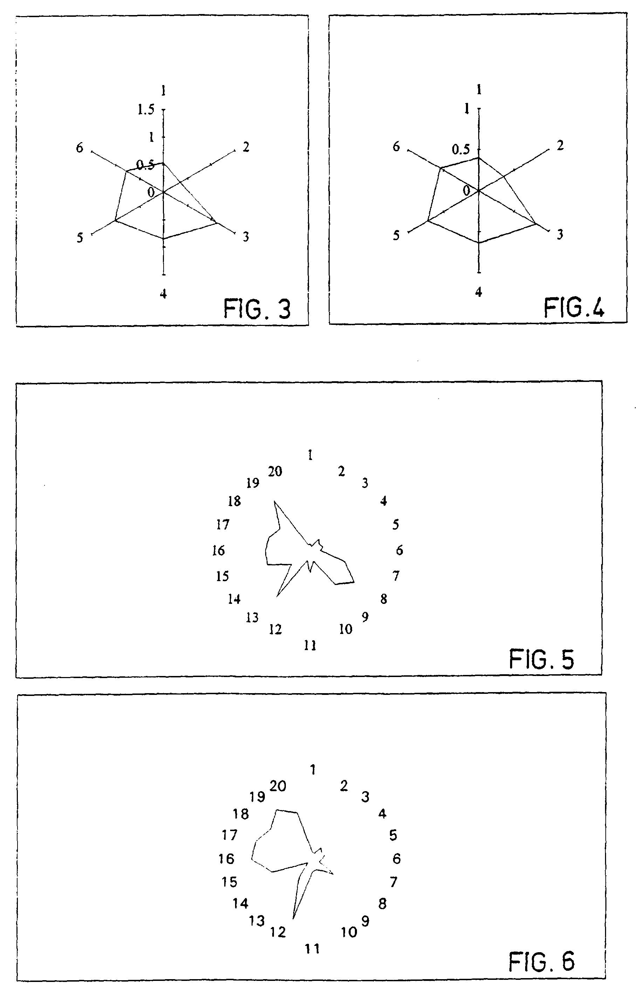

Plusieurs expériences de détection de substances odorantes ont été réalisées (une partie d'entre elles suivant des procédés et avec des appareils selon l'invention), dont certaines, parmi les plus représentatives vont être maintenant décrites, avec l'aide des Figures 3 à 16.

Une première expérience se rapporte à la détection et la comparaison d'arômes de café, illustrée par les Figures 3 à 6.

Les Figures 3 et 4 qui ne font pas partie de l'invention, représentent respectivement les empreintes olfactives d'un café du Brésil et d'un café du Zaïre, données chacune par un ensemble de six capteurs à semiconducteur. Les empreintes sont présentées sous la forme de représentation en étoile, chaque branche correspondant à un capteur. On remarque que la discrimination des deux cafés par leurs empreintes respectives n'est pas évidente, compte tenu d'une sélectivité moyenne de ce type de capteurs.

Les Figures 5 et 6 selon l'invention représentent respectivement les empreintes olfactives des mêmes cafés du Brésil et du Zaïre, données chacune par un ensemble de six capteurs à semiconducteur et quatorze capteurs à polymère conducteur. Les empreintes sont également présentées sous la forme de représentation en étoile, afin de faciliter la comparaison avec les Figures 3 et 4. Les références de 1 à 6 se rapportent aux capteurs à semiconducteur, et les références 7 à 20 se rapportent aux capteurs à polymère conducteur.

On constate que les empreintes obtenues avec un tel ensemble de capteurs permettent de discriminer aisément les deux cafés, la partie déterminante de l'empreinte pour la différenciation des cafés étant essentiellement constituée par les capteurs à polymère conducteur.

Une deuxième expérience selon l'invention a été réalisée avec un premier parfum et un deuxième parfum différent du premier, illustrée par les Figures 7 et 8.

La Figure 7 représente une empreinte olfactive fonction du temps du premier parfum imprégné sur une serviette dans une chambre d'échantillons chauffée à 40°C. Une enceinte comprenant six capteurs à semiconducteur et une enceinte comprenant sept capteurs à polymère conducteur, montées en série, ont été utilisées pour cette expérience, qui s'est effectuée durant cinq jours avec un débit d'air de 10 ml/mn. Préalablement, les propriétés électriques des capteurs ont été testées durant dix heures en présence de la serviette seule et avec un débit de 10 ml/mn afin d'obtenir une initialisation des capteurs. La droite des abscisses est graduée en heures, et la droite des ordonnées est graduée en volts. La réponse significative de trois capteurs est représentée sur la Figure 7.

La Figure 8 représente une empreinte olfactive fonction du temps du deuxième parfum imprégné sur une serviette placée dans une chambre d'échantillons. Les conditions d'expérience sont identiques à celles concernant le premier parfum, de façon à pouvoir comparer les deux parfums.

La comparaison des Figures 7 et 8 montre que le parfum utilisé dans la Figure 8 a une note plus forte et plus stable dans le temps que le parfum utilisé dans la Figure 8, dont l'effet est totalement épuisé au bout de trois jours environ.

Une autre expérience qui ne fait pas partie de l'invention a eté réalisée avec un vin de table et de l'éthanol, illustrée par les Figures 9, 10 et 11.

Les Figures 9, 10 et 11 montrent des histogrammes, représentant les empreintes olfactives provenant respectivement d'un vin de table, de l'éthanol, et du bouquet dudit vin de table obtenu au moyen de l'unité de traitement de données par différence des empreintes olfactives du vin et de l'éthanol. L'expérience a été réalisée avec une enceinte munie de six capteurs à semiconducteur, un débit d'air faible, et les empreintes stabilisées ont été obtenues au bout de trente secondes environ. Chacun des six rectangles, référencés de 1 à 6 sur chaque Figure 9 et 10 représente respectivement la mesure d'un même capteur, au bout de trente secondes environ.

Une troisième expérience selon l'invention a été réalisée pour reconnaître la formulation originale de silicones et de polymères au moyen de deux enceintes munies respectivement chacune de six capteurs à semiconducteur et quatre capteurs à onde acoustique de surface. Les solvants résiduels de composés polymères tels que les polypropylènes étant à l'origine de certaines mauvaises odeurs de matériaux plastiques, un ensemble de capteurs de technologies différentes permet de détecter les écarts par rapport à une odeur de référence.

Une quatrième expérience selon l'invention concerne la détection et la reconnaissance des drogues.

Les drogues comme la morphine, la cocaïne ou autres produits illicites sont détectées en utilisant un ensemble de capteurs comprenant des capteurs à semiconducteur, des capteurs à polymère conducteur, et des capteurs à onde acoustique de surface. Lorsque les capteurs à onde acoustique de surface sont utilisés seuls avec un nombre de quatre capteurs par exemple, le taux de reconnaissance entre les différentes drogues analysées est de 90% (nombre de drogues reconnues / nombre de drogues analysées). Toutefois, dans ce dernier cas, ce taux de reconnaissance diminue jusqu'à 40% lorsqu'on étudie ces drogues mélangées avec des produits classiques comme le café, les tabacs, et autres poudres ou produits divers de consommation courante. Il est donc nécessaire d'utiliser d'autres technologies de capteurs qui permettent d'obtenir une sélectivité plus grande afin de présenter une empreinte olfactive des drogues, détectable même en présence de plusieurs substances différentes. L'addition aux quatre capteurs à onde acoustique de surface, de six capteurs à semiconducteur permet d'obtenir un taux de reconnaissance de 65%, et l'addition supplémentaire de 14 capteurs à polymère conducteur permet d'augmenter le taux de reconnaissance à une valeur de 85%.

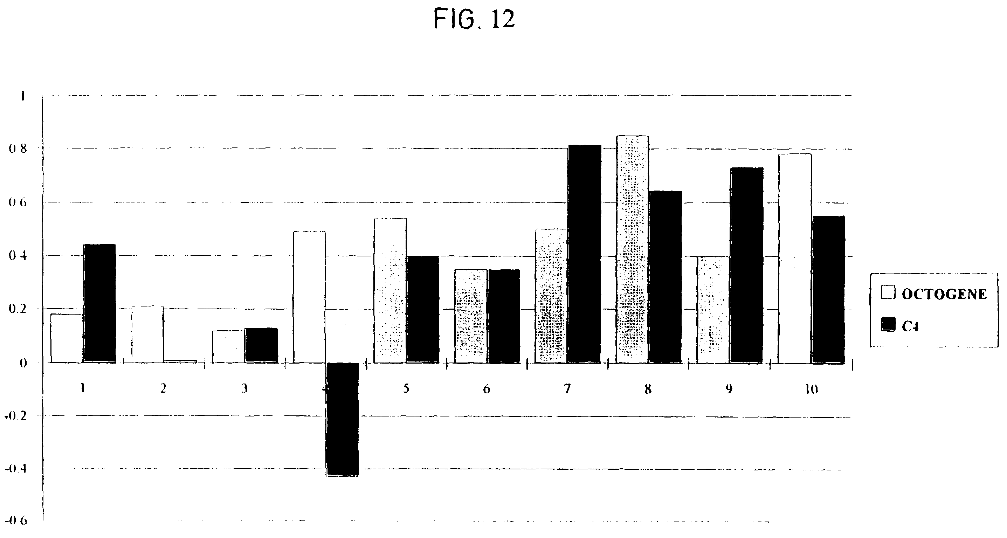

Une cinquième expérience selon l'invention concerne la détection et la reconnaissance des explosifs, illustrée par les Figures 12, 13 et 14.

La Figure 12 représente les empreintes olfactives de deux explosifs différents, sous forme d'histogramme. Les rectangles foncés représentent un explosif de type C4 et les rectangles clairs représentent un explosif de type Octogène (HMX). Les valeurs de chaque capteur pour chacun des explosifs sont représentées côte à côte pour faciliter une comparaison. Pour réaliser cette expérience, on a utilisé un ensemble de quatre capteurs à onde acoustique de surface et six capteurs à semiconducteur. Les empreintes olfactives des explosifs ont été préalablement enregistrées dans la mémoire de l'unité de traitement de données, ce qui permet d'identifier et de reconnaître ultérieurement un explosif par ses substances volailles. Sur la droite des abscisses, les références de 1 à 4 se rapportent aux capteurs à onde acoustique de surface, et les références de 5 à 10 se rapportent aux capteurs à semiconducteur. Il est à noter que la variation de fréquence du capteur de référence 4, due à un effet viscoélastique caractéristique du revêtement de ce capteur, constitue une partie importante de l'empreinte visant à discriminer l'explosif C4 de l'explosif Octogène.

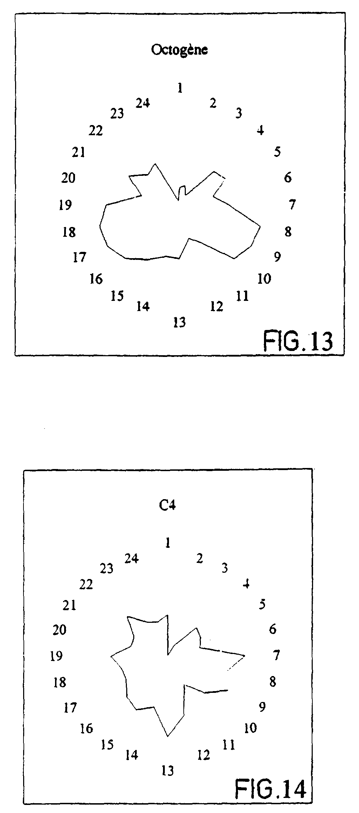

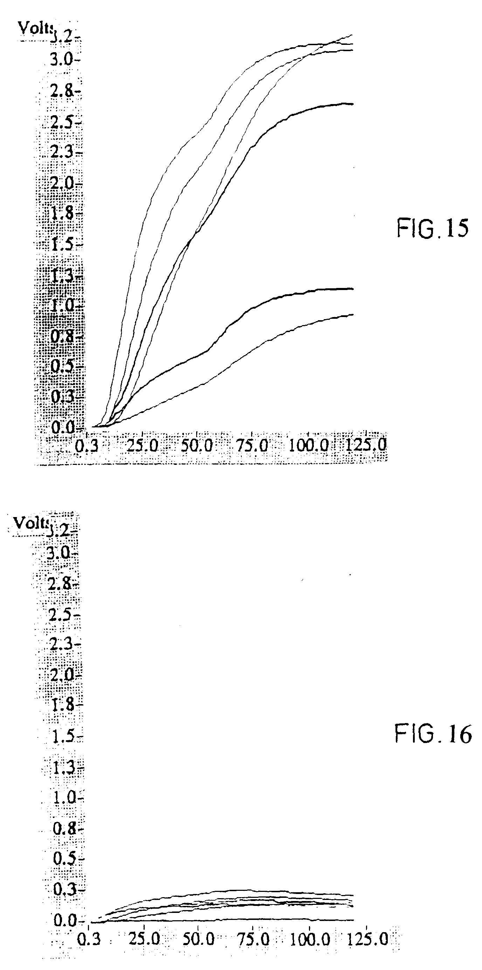

Les Figures 13 et 14 représentent les empreintes olfactives des mêmes explosifs, respectivement Octogène et C4. Ces empreintes ont été obtenues avec un ensemble de capteurs similaires à ceux utilisés sur la Figure 12, ensemble auquel ont été ajoutés quatorze capteurs à polymère conducteur, soit un nombre total de vingt quatre capteurs. Sur les Figures 13 et 14, les diagrammes sont représentés sous forme d'étoile, les références 1 à 4 se rapportant aux capteurs à onde acoustique de surface, les références 5 à 10 se rapportant aux capteurs à semiconducteur, et les références 11 à 24 se rapportant aux capteurs à polymère conducteur. On constate une nette différenciation des empreintes, caractéristiques de chaque explosif.

Une autre expérience qui ne fait pas partie de l'invention concerne la détection et la reconnaissance des odeurs de bouchons alimentaires en liège, illustrée par les Figures 15 et 16.

Un premier et un deuxième bouchon ont été placés respectivement dans une première et une deuxième chambre d'échantillons de 60 ml chacune et chauffées à une température de 60°C durant 5 mn. Les vapeurs des deux bouchons sont ensuite transportées successivement dans une enceinte comportant six capteurs à semiconducteur. Les Figures 15 et 16 représentent respectivement les empreintes olfactives fonction du temps obtenues pour le premier bouchon et le deuxième bouchon, pendant 120 secondes. On constate une nette différence des empreintes montrant que le premier bouchon est impropre à l'utilisation ou présente un risque certain de contamination du produit enfermé par ledit bouchon, par une intensité d'odeurs plus forte et des formes d'empreinte caractéristiques des mauvaises odeurs de bouchons. Le deuxième bouchon présente une empreinte standard correcte et est apte à l'utilisation. On notera avantageusement que le procédé permet dans ce cas d'établir une sélection olfactive des bouchons lors de leur fabrication et évite ainsi les pertes de produits bouchés avec des bouchons défectueux et contaminés par ceux-ci, comme notamment les vins et spiritueux.

Une sixième expérience selon l'invention concerne la détection et la reconnaissance des odeurs corporelles humaines.

Les odeurs corporelles sont analysées en plaçant un sujet dans une première chambre traversée par un débit d'air pur. L'air chargé des substances odorantes dégagées par le sujet traverse ensuite avantageusement une deuxième chambre substantiellement moins volumineuse, qui sert de concentrateur des composés volatils. Cette deuxième chambre peut être constituée d'un piège Ténax ou autres supports polymères absorbant. Par un réchauffage très rapide de la deuxième chambre après ladite concentration des composés volatils, le débit d'air chargé des substances odorantes du sujet est envoyé sur un ensemble de capteurs, lequel peut avantageusement comprendre une pluralité de capteurs à semiconducteur, une pluralité de capteurs à onde acoustique de surface, et une pluralité de capteurs à polymère conducteur. Les données de mesure des capteurs sont simultanément comparées à un fichier de substances odorantes de criminels ou d'individus déjà incriminés afin de reconnaître le sujet.

Il faut mentionner que la deuxième chambre n'est pas indispensable au procédé de détection et de reconnaissance des odeurs corporelles selon l'invention. En effet, l'air chargé des substances odorantes dégagées par le sujet dans la première chambre peut directement être envoyé sur les capteurs, l'utilisation d'un concentrateur ayant pour objectif notamment d'améliorer la qualité et la rapidité d'obtention de l'empreinte olfactive.

Une autre expérience concerne la détection et la reconnaissance des odeurs corporelles animales.

Un appareil qui ne fait pas partie de l'invention comprenant une pluralité de capteurs à polymère conducteur a été placé de façon à détecter les substances odorantes émanant des parties génitales d'une jument. On obtient des empreintes olfactives caractéristiques de la période de fécondation de l'animal. Des empreintes olfactives caractéristiques de la période de fécondation d'un animal ont été obtenues de façon similaire en suivant les périodes de fertilité sur une vache et sur une chienne.

Il est possible de procéder à un test de la fertilité de la vache en mesurant les vapeurs émises par son lait. La période de fécondation induit ainsi sur le lait des composés volatils responsables d'une série de réponses caractéristiques de la fertilité de l'animal.

Comme on peut le constater, le champ des applications des procédés et appareils de détection des substances odorantes selon l'invention est très étendu, les expériences décrites ci-dessus n'illustrant que quelques exemples dans ce champ d'application.

On peut citer encore à titre d'exemples non limitatifs les applications des procédés et appareils selon l'invention suivantes :
- détection et mesure de l'efficacité de déodorants et des odeurs bactériennes par connexion directe d'un appareil selon l'invention sous les aisselles de différents sujets,
- évaluation du caractère plaisant ou déplaisant de l'odeur,
- diagnostic de maladies à partir de l'odeur corporelle,
- discrimination aromatique de qualités de grains et mélanges de café, suivi de processus de torréfaction,
- distinction de différentes qualités olfactives d'huiles d'olives (odeurs rances, fruitées ...), conséquentes à des conditions climatologiques ou des traitements particuliers,
- mesure en continue d'atmosphères closes. Contrôle d'ambiances enfumées avec seuil d'alarme éventuel (ou contrôle de ventilation) à partir d'un seuil prédéterminé d'un niveau d pollution,
- mesure de l'intensité et de la qualité de l'odeur de granules de polyéthylène, de PVC, de silicones et autres matériaux plastiques,
- contrôle de l'odeur sur les matériaux plastiques finis et évaluation de la contribution individuelle olfactive de chaque composant à l'odeur globale d'un habitacle (intérieur de voitures, de navettes ...),
- contrôle des hydrocarbures et odeurs résiduelles à l'intérieur de bouteilles en plastique,
- à partir d'une viande, discrimination du sexe de l'animal d'origine.

## Revendications

1. Procédé de détection des substances odorantes ou volatiles comprenant les étapes suivantes :
- munir au moins une première et une deuxième enceinte respectivement de premiers et deuxièmes moyens de détection, lesdits premiers et deuxièmes moyens de détection possédant des propriétés électriques ou piézo-électriques sensibles aux molécules desdites substances odorantes ou volatiles,
- tester les propriétés électriques ou piézo-électriques de chacun desdits premiers et deuxièmes moyens de détection, ceux-ci étant en contact avec un débit variable contrôlé de gaz, pour effectuer une initialisation desdits premiers et deuxièmes moyens de détection avant la présence desdites substances odorantes ou volatiles,
- transporter lesdites substances odorantes ou volatiles d'un échantillon dans lesdites première et deuxième enceintes au moins, dans ledit débit variable contrôlé de gaz,
- mesurer les propriétés électriques ou piézo-électriques, pendant un intervalle de temps déterminé, de chacun desdits premiers et deuxièmes moyens de détection en présence dudit débit variable contrôlé de gaz comportant lesdites substances odorantes ou volatiles dudit échantillon, relativement à ladite initialisation, pour obtenir des données qualitatives et quantitatives sur lesdites substances odorantes ou volatiles dudit échantillon,
- comparer lesdites données au moins qualitatives dudit échantillon avec un fichier de substances odorantes ou volatiles, simultanément ou postérieurement aux mesures elles-mêmes, de manière à au moins identifier les substances odorantes ou volatiles détectées,
caractérisé en ce qu'il comprend au moins les étapes suivantes :
- munir lesdits premiers moyens de détection de ladite première enceinte (1) d'une pluralité de capteurs gaz à semiconducteur (6), ou d'une pluralité de capteurs gaz à polymère conducteur (7), ou d'une pluralité de capteurs gaz à onde acoustique de surface (8),
- munir lesdits deuxièmes moyens de détection de ladite deuxième enceinte (2) d'une pluralité de capteurs gaz à semiconducteur (6), ou d'une pluralité de capteurs gaz à polymère conducteur (7), ou d'une pluralité de capteurs gaz à onde acoustique de surface (8), lesdits premiers et deuxièmes moyens de détection étant munis respectivement de capteurs gaz de types de technologie différentes,
- nettoyer lesdits premiers et deuxièmes moyens de détection par un balayage de ces derniers avec un gaz, pour initialiser lesdits premiers et deuxièmes moyens de détection.

2. Procédé suivant la revendication 1, caractérisé en ce qu'il consiste à munir une troisième enceinte (3) de troisièmes moyens de détection, lesdits troisièmes moyens de détection comprenant une pluralité de capteurs gaz à semiconducteur (6), ou une pluralité de capteurs gaz à polymère conducteur (7), ou une pluralité de capteurs gaz à onde acoustique de surface (8).

3. Procédé suivant la revendication 1 ou 2, caractérisé en ce que lesdites première (1) et deuxième (2) enceintes au moins, ou première (1) et deuxième (2) et troisième (3) enceintes au moins, sont placées en série.

4. Procédé suivant la revendication 1 ou 2, caractérisé en ce que lesdites première (1) et deuxième (2) enceintes au moins, ou première (1) et deuxième (2) et troisième (3) enceintes au moins, sont placées en parallèle.

5. Procédé suivant l'une quelconque des revendications 1 à 4, caractérisé en ce qu'il comporte en outre l'étape de séparer lesdites substances odorantes ou volatiles au moyen d'un dispositif de chromatographie gazeuse.

6. Procédé selon l'une quelconque des revendications 1 à 5, caractérisé en ce que l'étape de comparaison des données au moins qualitatives dudit échantillon avec un fichier de substance odorante ou volatile s'effectue en utilisant un moyen de réseau neuronal.

7. Procédé selon l'une quelconque des revendications 1 à 6, caractérisé en ce que le débit de gaz est modifié pendant la phase de mesure.

8. Utilisation du procédé suivant l'une quelconque des revendications 1 à 7, pour les drogues et/ou explosifs, pour permettre une détection et/ou une reconnaissance desdits drogues et/ou explosifs.

9. Utilisation du procédé suivant l'une quelconque des revendications 1 à 7, pour les odeurs corporelles humaines, pour permettre une identification et/ou une reconnaissance des individus.

10. Utilisation du procédé suivant l'une quelconque des revendications 1 à 7, pour les odeurs corporelles humaines, pour permettre un diagnostic des maladies humaines.

11. Utilisation du procédé suivant l'une quelconque des revendications 1 à 7, pour les odeurs animales, pour permettre une détermination de la période de fertilité desdits animaux et/ou pour permettre un diagnostic des maladies desdits animaux.

12. Utilisation du procédé suivant l'une quelconque des revendications 1 à 7, pour les bouchons alimentaires, pour permettre une discrimination et/ou une sélection qualitative desdits bouchons.

13. Utilisation du procédé suivant l'une quelconque des revendications 1 à 7, pour les substances alimentaires, pour permettre une discrimination et/ou une comparaison qualitative et/ou un diagnostic de comestibilité, desdites substances alimentaires.

14. Utilisation du procédé suivant l'une quelconque des revendications 1 à 7, pour les parfums et/ou cosmétiques, pour permettre une appréciation des caractéristiques desdits parfums et/ou cosmétiques.

15. Utilisation du procédé suivant l'une quelconque des revendications 1 à 7, pour les substances toxiques, pour permettre une détermination et/ou une surveillance d'un niveau de pollution.

16. Utilisation du procédé suivant l'une quelconque des revendications 1 à 7, pour les matériaux plastiques, pour permettre une évaluation d'une contribution olfactive individuelle de chaque composant desdits matériaux plastiques à l'odeur globale desdits matériaux plastiques, et/ou pour un contrôle de l'odeur globale desdits matériaux plastiques.

17. Appareil de détection des substances odorantes ou volatiles, permettant de mettre en oeuvre un procédé suivant l'une des revendications 1 à 5, comprenant au moins une première et une deuxième enceinte munies respectivement de premiers et deuxièmes moyens de détection possédant des propriétés électriques ou piézo-électriques sensibles aux molécules desdites substances odorantes ou volatiles, des moyens de pompe (10) à débit variable, des moyens de test et de mesure (15) des propriétés électriques ou piézo-électriques de chacun desdits premiers et deuxièmes moyens de détection pour obtenir des données qualitatives et quantitatives sur lesdites substances odorantes ou volatiles, et des moyens de comparaison (16) desdites données au moins qualitatives avec un fichier de substances odorantes ou volatiles, pour au moins identifier lesdites substances odorantes ou volatiles caractérisé en ce que lesdits premiers moyens de détection de ladite première enceinte (1) comportent une pluralité de capteurs gaz à semiconducteur (6), ou une pluralité de capteurs gaz à polymère conducteur (7), ou une pluralité de capteurs gaz à onde acoustique de surface (8), en ce que lesdits deuxièmes moyens de détection de ladite deuxième enceinte (2) comportent une pluralité de capteurs gaz à semiconducteur (6), ou une pluralité de capteurs gaz à polymère conducteur (7), ou une pluralité de capteurs gaz à onde acoustique de surface (8), lesdits premiers et deuxièmes moyens de détection étant munis respectivement de capteurs gaz de types de technologie différentes.

18. Appareil selon la revendication 17, caractérisé en ce que les moyens de comparaison (16) des données au moins qualitatives de l'échantillon avec un fichier de substance odorante ou volatile comprennent un moyen de réseau neuronal.

19. Appareil selon la revendication 17 ou 18, caractérisé en ce que les moyens de pompe (10) à débit variable sont adaptés à varier le débit de gaz pendant la phase de mesure.

## Patentansprüche

1. Verfahren zum Nachweisen duftender oder flüchtiger Substanzen, umfassend folgende Schritte:
- mindestens eine erste und eine zweite Kammer werden mit einer ersten bzw. einer zweiten Nachweiseinrichtung ausgerüstet, wobei die erste und die zweite Nachweiseinrichtung elektrische oder piezoelektrische Eigenschaften besitzen, die empfindlich gegenüber Molekülen der erwähnten duftenden oder flüchtigen Substanzen sind,
- Prüfen der elektrischen oder piezoelektrischen Eigenschaften der ersten und der zweiten Nachweiseinrichtung, wobei diese in Berührung stehen mit einer variabel gesteuerten Gasdurchsatzmenge, um eine Initialisierung der ersten und der zweiten Nachweiseinrichtung vor dem Vorhandensein der duftenden oder flüchtigen Substanzen vorzunehmen,
- Transportieren der duftenden oder flüchtigen Substanzen einer Probe in zumindest die erste und die zweite Kammer innerhalb der variabel gesteuerten Gasdurchsatzmenge,
- Messen der elektrischen oder piezoelektrischen Eigenschaften während eines bestimmten Zeitintervalls sowohl der ersten als auch der zweiten Nachweiseinrichtung im Beisein der variabel gesteuerten Gasdurchsatzmenge, welche die duftenden oder flüchtigen Substanzen der Probe beinhaltet, relativ bezüglich der erwähnten Initialisierung, um qualitative und quantitative Daten über die duftenden oder flüchtigen Substanzen der Probe zu gewinnen,
- Vergleichen dieser zumindest qualitativen Daten der Probe mit einer Datei von duftenden oder flüchtigen Substanzen entweder gleichzeitig mit oder nach den Messungen, um dadurch die nachgewiesenen duftenden oder flüchtigen Substanzen zumindest zu identifizieren,
dadurch gekennzeichnet, daß das Verfahren mindestens die folgenden Schritte beinhaltet:
- Ausstatten der ersten Nachweiseinrichtung der ersten Kammer (1) mit mehreren Halbleiter-Gassensoren (6) oder mehreren Polymerleiter-Gassensoren (7) oder mehreren mit akustischen Oberflächenwellen arbeitenden Gassensoren (8),
- Ausstatten der zweiten Nachweiseinrichtung der zweiten Kammer (2) mit mehreren Halbleiter-Gassensoren (6) oder mehreren Polymerleiter-Gassensoren (7) oder mehreren mit akustischen Oberflächenwellen arbeitenden Gassensoren (8), wobei die erste und die zweite Nachweiseinrichtung mit Gassensor-Typen verschiedener Technologie ausgestattet sind,
- Reinigen der ersten und der zweiten Nachweiseinrichtung, indem sie mit einem Gas gespült werden, um die erste und die zweite Nachweiseinrichtung zu initialisieren.

2. Verfahren nach Anspruch 1, dadurch gekennzeichnet, daß eine dritte Kammer (3) mit einer dritten Nachweiseinrichtung vorgesehen wird, wobei die dritte Nachweiseinrichtung mehrere Halbleiter-Gassensoren (6) oder mehrere Polymerleiter-Gassensoren (7) oder mehrere mit akustischen Oberflächenwellen arbeitende Gassensoren (8) aufweist.

3. Verfahren nach Anspruch 1 oder 2, dadurch gekennzeichnet, daß zumindest die erste (1) und die zweite Kammer (2) oder zumindest die erste (1) und die zweite (2) sowie die dritte (3) Kammer in Reihe geschaltet sind.

4. Verfahren nach Anspruch 1 oder 2, dadurch gekennzeichnet, daß zumindest die erste (1) und die zweite Kammer (2) oder zumindest die erste (1), die zweite (2) und die dritte (3) Kammer parallel geschaltet sind.

5. Verfahren nach einem der Ansprüche 1 bis 4, dadurch gekennzeichnet, daß es außerdem den Schritt des Separierens der duftenden oder flüchtigen Substanzen mit Hilfe eines Gaschromatographen beinhaltet.

6. Verfahren nach einem der Ansprüche 1 bis 5, dadurch gekennzeichnet, daß der Schritt des Vergleichens der zumindest qualitativen Daten der Probe mit einer Datei der duftenden oder flüchtigen Substanz unter Einsatz eines neuronalen Netzwerks erfolgt.

7. Verfahren nach einem der Ansprüche 1 bis 6, dadurch gekennzeichnet, daß der Gasdurchsatz während der Meßphase modifiziert wird.

8. Verwendung des Verfahrens nach einem der Ansprüche 1 bis 7 für Drogen und/oder Sprengstoffe, um einen Nachweis und/oder ein Erkennen der Drogen und/oder Sprengstoffe zu ermöglichen.

9. Verwendung des Verfahrens nach einem der Ansprüche 1 bis 7 für menschliche Körpergerüche, um eine Identifizierung und/oder eine Erkennung von Personen zu ermöglichen.

10. Verwendung des Verfahrens nach einem der Ansprüche 1 bis 7 für menschliche Körpergerüche, um eine Diagnose von Humankrankheiten zu ermöglichen.

11. Verwendung des Verfahrens nach einem der Ansprüche 1 bis 7 für tierische Gerüche, um eine Feststellung der Fruchtbarkeitszeitspanne der Tiere zu ermöglichen und/oder eine Diagnose von Krankheiten der Tiere zu ermöglichen.

12. Verwendung des Verfahrens nach einem der Ansprüche 1 bis 7 für Nahrungsverschlüsse, um eine Unterscheidung und/oder eine qualitative Auswahl der Verschlüsse zu ermöglichen.

13. Verwendung des Verfahrens nach einem der Ansprüche 1 bis 7 für Nahrungsmittel, um eine Unterscheidung und/oder einen qualitativen Vergleich und/oder eine Diagnose der Nahrhaftigkeit dieser Nahrungsmittel zu ermöglichen.

14. Verwendung des Verfahrens nach einem der Ansprüche 1 bis 7 für Parfüms und/oder Kosmetika, um eine Bewertung der Eigenschaften dieser Parfüms und/oder Kosmetika zu ermöglichen.

15. Verwendung des Verfahrens nach einem der Ansprüche 1 bis 7 für toxische Substanzen, um eine Feststellung und/oder eine Überwachung eines Verschmutzungsgrads zu ermöglichen.

16. Verwendung des Verfahrens nach einem der Ansprüche 1 bis 7 für Kunststoffe, um eine Ermittlung des individuellen olfaktorischen Beitrags jedes Bestandteils dieser Kunststoffe zu dem Gesamtgeruch dieser Kunststoffe zu ermöglichen, und/oder eine Kontrolle des gesamten Geruchs dieser Kunststoffe zu gestatten.

17. Vorrichtung zum Nachweisen von duftenden oder flüchtigen Substanzen, geeignet zur Ausführung des Verfahrens nach einem der Ansprüche 1 bis 5, umfassend mindestens eine erste und eine zweite Kammer, die mit einer ersten bzw. einer zweiten Nachweiseinrichtung ausgestattet sind, welche elektrische oder piezoelektrische Eigenschaften aufweisen, die bezüglich Molekülen der duftenden oder flüchtigen Substanzen empfindlich sind, eine Pumpeneinrichtung (10) mit veränderlichem Durchsatz, eine Einrichtung zum Testen und zum Messen (15) der elektrischen oder piezoelektrischen Eigenschaften sowohl der ersten als auch der zweiten Nachweiseinrichtung, um qualitative und quantitative Daten über die duftenden oder flüchtigen Substanzen zu gewinnen, und eine Vergleicheinrichtung (16) zum Vergleichen der zumindest qualitativen Daten mit einer Datei von duftenden oder flüchtigen Substanzen, um die duftenden oder flüchtigen Substanzen zumindest zu identifizieren, dadurch gekennzeichnet, daß die erste Nachweiseinrichtung der ersten Kammer (1) eine Mehrzahl von Halbleiter-Gassensoren (6) oder eine Mehrzahl von Polymerleiter-Gassensoren (7) oder eine Mehrzahl von mit akustischen Oberflächenwellen arbeitenden Gassensoren (8) enthält, daß die zweite Nachweiseinrichtung der zweiten Kammer (2) eine Mehrzahl von Halbleiter-Gassensoren (6) oder eine Mehrzahl von Polymerleiter-Gassensoren (7) oder eine Mehrzahl von mit akustischen Oberflächenwellen arbeitenden Gassensoren (8) aufweist, wobei die erste und die zweite Nachweiseinrichtung mit Gassensoren unterschiedlicher Technologie ausgestattet sind.

18. Vorrichtung nach Anspruch 17, dadurch gekenzeichnet, daß die Vergleichereinrichtung (16) zum Vergleichen der zumindest qualitativen Daten der Probe mit einer Datei der duftenden oder flüchtigen Substanz eine neuronale Netzwerkeinrichtung aufweist.

19. Vorrichtung nach Anspruch 17 oder 18, dadurch gekennzeichnet, daß die Pumpeneinrichtung (10) mit variablem Durchsatz dazu ausgebildet ist, den Gasdurchsatz während der Meßphase zu variieren.

## Claims

1. A method of detecting odorous or volatile substances, comprising the following steps:
- providing at least one first enclosure and one second enclosure respectively with first and second detection means, the said first and second detection means having electric or piezoelectric properties sensitive to the molecules of the said odorous or volatile substances,
- testing the electric or piezoelectric properties of each of the said first and second detection means, the latter being in contact with a controlled variable gas flow in order to effect initialisation of said first and second detection means before the presence of said odorous or volatile substances,
- transporting the said odorous or volatile substances of a sample into the said at least first and second enclosures in the controlled variable gas flow,
- measuring the electric or piezoelectric properties, for a predetermined interval of time, of each of the said first and second detection means in the presence of the said controlled variable gas flow containing the said odorous or volatile substances of the said sample, relatively to the said initialisation, in order to obtain qualitative and quantitative data concerning the said odorous or volatile substances of the said sample,
- comparing the said at least qualitative data of the said sample with an odorous or volatile substance file, simultaneously with or subsequently to the actual measurements, so as to at least identify the odorous or volatile substances detected,
characterised in that it comprises at least the following steps:
- providing the said first detection means of the said first enclosure (1) with a plurality of semiconductor type gas sensors (6), or a plurality of conductive polymer type gas sensors (7) or a plurality of acoustic surface wave type gas sensors (8),
- providing the said second detection means of the said second enclosure (2) with a plurality of semiconductor type gas sensors (6), or a plurality of conductive polymer type gas sensors (7), or a plurality of acoustic surface wave type gas sensors (8), the said first and second detection means being respectively provided with gas sensors of different technological types,
- cleaning the said first and second detection means by scavenging the same with a gas, in order to initialise the said first and second detection means.

2. A method according to claim 1, characterised in that it comprises providing a third enclosure (3) with third detection means, the said third detection means comprising a plurality of semiconductor type gas sensors (6) or a plurality of conductive polymer type gas sensors (7), or a plurality of acoustic surface wave type gas sensors (8).

3. A method according to claim 1 or 2, characterised in that the said first enclosure (1) and second enclosure (2) at least, or first enclosure (1) and second enclosure (2) and third enclosure (3) at least, are disposed in series.

4. A method according to claim 1 or 2, characterised in that the said first enclosure (1) and second enclosure (2) at least, or the said first enclosure (1) and second enclosure (2) and third enclosure (3) at least, are disposed in parallel.

5. A method according to any one of claims 1 to 4, characterised in that it also comprises the step of separating the said odorous or volatile substances by means of a gas chromatography apparatus.

6. A method according to any one of claims 1 to 5, characterised in that the step for comparison of the at least qualitative data of the said sample with an odorous or volatile substance file is effected by using a neuronal network means.

7. A method according to any one of claims 1 to 6, characterised in that the gas flow is modified during the measuring phase.

8. Use of the method according to any one of claims 1 to 7, for drugs and/or explosives, to allow detection and/or recognition of the said drugs and/or explosives.

9. Use of the method according to any one of claims 1 to 7, for human body odours, to allow identification and/or recognition of individuals.

10. Use of the method according to any one of claims 1 to 7, for human body odours, to allow diagnosis of human diseases.

11. Use of the method according to any one of claims 1 to 7, for animal odours, to allow the period of fertility of the said animals to be determined and/or to allow diagnosis of diseases of the said animals.

12. Use of the method according to any one of claims 1 to 7, for closures for foodstuffs, to allow discrimination and/or qualitative selection of the said closures.

13. Use of the method according to any one of claims 1 to 7, for food substances, to allow discrimination and/or qualitative comparison and/or diagnosis of edibility of said food substances.

14. Use of the method according to any one of claims 1 to 7, for perfumes and/or cosmetics, to allow assessment of the characteristics of the said perfumes and/or cosmetics.

15. Use of the method according to any one of claims 1 to 7, for toxic substances, to allow determination and/or supervision of a pollution level.

16. Use of the method according to any one of claims 1 to 7, for plastic materials, to allow evaluation of an individual olfactory contribution of each component of the said plastic materials to the overall odour of the said plastic materials, and/or for a monitoring of the overall odour of the said plastic materials.

17. Apparatus for detecting odorous or volatile substances, for performing a method according to any one of claims 1 to 5, comprising at least one first enclosure and one second enclosure respectively provided with first and second detection means having electric or piezoelectric properties sensitive to the molecules of the said odorous or volatile substances, variable flow pump means (10), means (15) for testing and measuring the electric or piezoelectric properties of each of the said first and second detection means to obtain qualitative and quantitative data concerning the said odorous or volatile substances, and means (16) for comparing said at least qualitative data with an odorous or volatile substance file, for at least identifying said odorous or volatile substances, characterised in that the said first detection means of the said first enclosure (1) comprise a plurality of semiconductor type gas sensors (6) or a plurality of conductive polymer type gas sensors (7) or a plurality of acoustic surface wave type gas sensors (8), in that the said second detection means of the said second enclosure (2) comprise a plurality of semiconductor type gas sensors (6), or a plurality of conductive polymer type gas sensors (7), or a plurality of acoustic surface wave type gas sensors (8), the said first and second detection means being respectively provided with gas sensors of different technological types.

18. Apparatus according to claim 17, characterised in that the means (16) for comparing the at least qualitative data of the sample with an odorous or volatile substance file comprise a neuronal network means.

19. Apparatus according to claim 17 or 18, characterised in that the variable flow pump means (10) are adapted to vary the gas flow during the measuring phase.
